# EUROPÄISCHE PATENTSCHRIFT

(11) **EP 3 496 649 B1**
(45) Veröffentlichungstag und Bekanntmachung des Hinweises auf die Patenterteilung: **30.12.2020**
(21) Anmeldenummer: 17749460.6
(22) Anmeldetag: 08.08.2017
(51) Int. Cl.: A61C 5/62, A61C 5/66, A61C 19/06, A61M 5/34

(54) **KARTUSCHE ZUM LAGERN, AUSGEBEN UND AUFTRAGEN EINER DENTALMASSE**
CARTRIDGE FOR STORING, DISCHARGING AND APPLYING A DENTAL COMPOUND
CARTOUCHE POUR ENTREPOSER, FAIRE SORTIR ET APPLIQUER UNE MASSE DENTAIRE

(30) Priorität: 12.08.2016 DE 202016004926 U
(43) Veröffentlichungstag der Anmeldung: 19.06.2019
(73) Patentinhaber: Mühlbauer Technology GmbH, 22547 Hamburg (DE)
(72) Erfinder: EFFENBERGER, Steffen, 20255 Hamburg (DE); NOWAK, Axel, 22299 Hamburg (DE)
(74) Vertreter: Glawe, Delfs, Moll
(86) Internationale Anmeldenummer: PCT/EP2017/070048
(87) Internationale Veröffentlichungsnummer: WO 2018/029185

(56) Entgegenhaltungen:
- DE-A1- 1 809 613
- DE-A1-102012 202 693
- JP-A- 2009 125 341
- US-A- 6 095 813
- US-A1- 2015 112 251

## Beschreibung

Die Erfindung betrifft eine Kartusche zum Lagern, Ausgeben und Auftragen einer Dentalmasse, insbesondere einer Paste zur Aufbereitung und Trockenlegung der Zahnfleischfurche (sulcus gingivae) im Rahmen einer dentalen Abdrucknahme.

Im Stand der Technik sind verschiedene Pasten zur Aufweitung der Zahnfleischfurche bekannt. Neben niedrig- und mittelviskosen Materialien sind auch hochviskose Materialen, insbesondere auf Basis von Tonmineralen, bekannt. Aufgrund ihrer Konsistenz und hohen Viskosität lassen sich entsprechende Pasten gegen den Widerstand des Zahnfleischs tief in die Zahnfleischfurche einbringen, um das Zahnfleisch vom Zahn wegzudrücken und über den erforderlichen Zeitraum wegzuhalten. Eine entsprechende Paste ist bspw. beschrieben in US 2012/077142 A1.

Zum Ausgeben und Auftragen hochviskoser Pasten sind im Stand der Technik bereits verschiedene Vorrichtungen und Systeme bekannt. Die dabei verwendeten Behälter für die Pasten enthalten regelmäßig nur eine Einmaldosis und müssen entsprechend preiswert und einfach hergestellt werden können. Gleichzeitig soll eine einfache, schnelle und sichere Anwendung gewährleistet sein. Insbesondere muss die Paste bei Raumtemperatur mindestens 6 Monate, bevorzugt 12 oder 24 Monate gelagert werden können, ohne dass die sichere Anwendung gefährdet wäre. Insbesondere dürfen sich die Materialeigenschaften der Paste nicht verändern.

Zur sicheren Anwendung von Vorrichtungen zum Ausgeben und Auftragen hochviskoser Pasten im Bereich der Zahnmedizin ist es weiterhin erforderlich, die Auspresskräfte möglichst gering zu halten. Gleichzeitig müssen die verwendeten Behältern den beim Auspressen entstehenden Drücken standhalten.

Aus der US-A-8,616,879 ist ein System zum Ausbringen einer Paste bekannt, bei dem ein zylindrischer Behälter an der einen Seite durch einen Kolben und auf der anderen Seite von einer entfernbaren Kappe verschlossen wird. Vor der Anwendung muss die Kappe entfernt und über eine Schraubverbindung eine Metallkanüle an dem Behälter befestigt werden. Die Verwendung eines entsprechenden Systems erfordert mehrere Handgriffe. Außerdem sind bei Systemen mit Schraubverbindung die Herstellungskosten hoch.

Aus der US-A-6,095,814 sowie der US-B2-8,882,502 sind Vorrichtungen zum Ausbringen von hochviskosen Pasten bekannt, bei denen an einem zylindrischen Behälter unmittelbar eine steife Kanüle angeformt ist. Der Behälter und die Kanüle sind dabei aus Kunststoff gefertigt. Um die letztendliche Verwendung des Behälters mit daran angeformter Kanüle zu erleichtern, ist die Kanüle vorzugsweise in einem Winkel zur Achse des zylindrischen Behälters - bspw. 45° in US-B2-8,882,502 oder 60° in US- A-6,095,814 - angeordnet. Aufgrund der Anordnung der Kanüle in einem Winkel gegenüber dem zylindrischen Behälter ist die Herstellung der entsprechenden Vorrichtungen aufwendig. Aus DE 10 2012 202693 A1 ist eine Kartusche mit den Merkmalen des Oberbegriffs des Anspruchs 1 bekannt. Dieses Dokument offenbart weiterhin in getrennten Ausführungsvarianten eine dauerhaft verbiegbare Metallkanüle und eine flexibel verformbare Kunststoffkanüle, wobei in beiden Fällen die Kanüle getrennt vom Behälter ausgeführt und mit diesem stoffschlüssig verbunden ist.

Der Erfindung liegt die Aufgabe zugrunde, eine Kartusche zum Lagern, Ausgeben und Auftragen einer Dentalmasse zu schaffen, bei der die Nachteile aus dem Stand der Technik nicht mehr oder nur noch in vermindertem Umfang auftreten.

Gelöst wird diese Aufgabe durch eine Kartusche gemäß dem unabhängigen Anspruch 1. Vorteilhafte Weiterbildungen sind Gegenstand der abhängigen Ansprüche.

Zunächst werden einige im Rahmen der Erfindung verwendete Begriffe erläutert.

Eine Kanüle ist "unter manueller Kraftaufwendung" plastisch verformbar, wenn für die Verformung keine besonderen Werkzeuge o.ä. erforderlich sind, sondern sich im vorliegenden Fall die Biegung allein durch die Hände bzw. - aufgrund der typischen Größe für Kartuschen zum Lagern, Ausgeben und Auftragen von Dentalmassen - einzelne Finger erreichen lässt. Dabei ist von einer Person mit durchschnittlichem Leistungsvermögen auszugehen.

Bei einer "dauerhaften Biegung" handelt es sich um die Biegung aufgrund plastischer Verformung, die nach dem Wegfall der Biegekräfte - also der manuellen Kraftaufwendung - in der Kanüle bestehen bleibt. Der Winkel der Biegung entspricht dem Winkel zwischen den beiden Tangenten zu beiden Seiten der Biegung nach Wegfall der Biegekräfte.

Das Biegen der Kanüle erfolgt ohne Knicken, wenn die Krümmung der Kanüle nach dem Biegen stetig ist. Insbesondere tritt kein Beulen der Kanülenwand auf, durch welches der für das Durchtreten der Dentalmasse vorgesehene Innenquerschnitt der Kanüle verengt werden könnte. Die Änderung der Fläche des Innenquerschnitts - also des für den Durchfluss der Dentalmasse vorgesehenen Querschnitts - aufgrund der Biegung beträgt vorzugsweise höchstens 10%, vorzugsweise höchstens 5%.

Eine Paste gilt als "hochviskos", wenn sie bei einer Temperatur von 23°*C* eine dynamische Viskosität von über 27.000 *Pas* bei einer Scherspannung von 9.000 *N*/*m*₂ und einer Schergeschwindigkeit von 0,3¹/*ₛ* aufweist.

Die erfindungsgemäße Kartusche zeichnet sich durch einfache Herstellung sowie einfache und insbesondere flexible Verwendung aus. Indem sich die Kanüle in weiten Bereichen verbiegen lässt, kann ein Benutzer die Kanüle nach den jeweils vorliegenden Erfordernissen und seinen persönlichen Vorlieben anpassen. Auch lässt sich die Kanüle einfach herstellen, da sie im Ausgangszustand in gerader Richtung verlaufend ist.

Es ist erfindungsgemäß, wenn die Biegung der Kanüle beim Ausbringen der Dentalmasse aus der Hohlkammer mit einer Auspresskraft von weniger als 130 *N*, vorzugsweise von weniger als 150 *N*, weiter vorzugweise von weniger als 200 *N* nur um höchstens 10°, vorzugsweise um höchstens 5° zurückgestellt wird, weiter vorzugsweise um höchstens 1° zurückgestellt wird. Um dies zu erreichen, kann der Fachmann die Formgebung und/oder das Material der Kanüle auf die jeweilige Dentalmasse geeignet abstimmen. Eine entsprechende Abstimmung der Formgebung und/oder das Material der Kanüle ist in der Regel erforderlich, da die auf die gebogene Kanüle einwirkende Rückstellkraft neben der Auspresskraft maßgeblich von den Fließeigenschaften der Dentalmasse abhängt, stellt den Fachmann jedoch vor keine Probleme. Die Rückstellung beim Ausbringen der jeweiligen Dentalmasse lässt sich ebenfalls ohne weiteres durch entsprechende Erprobung feststellen.

Es ist bevorzugt, wenn der Kunststoff der Kanüle gemäß DIN EN ISO 178:2013 bei 23°*C* ein Biegemodul von 2,2 bis 2,9 *GPa,* vorzugsweise von 2,3 bis 2,6 *GPa,* vorzugsweise von ca. 2,4 *GPa* aufweist. Ein entsprechendes Biegemodul ist für die gewünschte Funktionsweise der Kanüle vorteilhaft.

Es ist bevorzugt, wenn die Auslassöffnung und die Aktuatoröffnung der Hohlkammer auf einer gemeinsamen Achse liegen. Weiter vorzugsweise verläuft auch die Kanüle im Ausgangszustand parallel zu oder entlang der Achse. Durch eine entsprechende Anordnung der Auslassöffnung und der Aktuatoröffnung der Hohlkammer, sowie ggf. der Kanüle im Ausgangszustand, ist eine einfache Herstellung der erfindungsgemäßen Kartusche möglich.

Die Kanüle ist erfindungsgemäß einstückig mit dem Behälter ausgebildet. In diesem Fall lässt sich die gesamte Kartusche in einem Arbeitsgang herstellen. Bspw. kann die Kanüle zusammen mit dem Behälter durch 1-Komponenten- oder Mehrkomponenten-Spritzgießen hergestellt werden.

Es ist erfindungsgemäß, wenn die Kanüle und/oder der Behälter aus einem thermoplastischen Kunststoff, vorzugsweise einem im Wesentlichen amorphen thermoplastischen Copolymer, Terpolymer und/oder Quadropolymer, weiter vorzugsweise einem teilvernetzten im Wesentlichen amorphen thermoplastischen Copolymer, Terpolymer oder Quadropolymer, weiter vorzugsweise aus Acrylnitril-Butadien-Styrol (ABS) ist. Entsprechende Materialien haben sich als grundsätzlich geeignet für die erfindungsgemäße Kanüle erwiesen.

Vorzugsweise enthält der thermoplastische Kunststoff Pigmente und/oder Füllstoffe, wobei der Anteil der Pigmente und/oder Füllstoffe vorzugsweise weniger als 50 Gewichts-%, weiter vorzugsweise weniger als 30 Gewichts-% beträgt.

Die erfindungsgemäße Kartusche ist vorzugsweise zur Verwendung mit einem Standarddispenser ausgebildet. Neben den dafür erforderlichen Abmaßen der Kartusche für einen Standarddispenser sind dadurch auch Maximalwerte für die vorgegebene Aktuator- und/oder Auspresskraft vorgegeben. Die Aktuator- und/oder Auspresskraft beträgt für einen Standarddispenser höchstens 200 *N*.

In der Hohlkammer ist ein Kolben vorgesehen, der im Ausgangszustand der Kartusche so angeordnet ist, dass die Dentalmasse in der Hohlkammer zwischen Kolben und Auslassöffnung aufgenommen werden kann. Indem ein entsprechender an die Hohlkammer angepasster Kolben vorgesehen ist, wird sichergestellt, dass eine in der Hohlkammer angeordnete Dentalmasse vollständig durch die Auslassöffnung ausgebracht werden kann. Insbesondere kann vermieden werden, dass Dentalmasse seitlich an dem Druckstempel eines Dispensers, der ggf. nicht an die Hohlkammer der Kartusche angepasst ist, vorbeidrängen und so an der Aktuatoröffnung der Kartusche austreten kann.

Es ist erfindungsgemäß, wenn die Auslassöffnung der Kanüle einen Innendurchmesser von 0,4 *mm* bis 2 *mm,* vorzugsweise 0,6 *mm* bis 0,9 *mm* aufweist, wobei das Auslassende der Kanüle vorzugsweise abgerundet ist, bevorzugt mit einem Radius von 0,5 *mm* bis 1,5 *mm*. Ein entsprechender Innendurchmesser gestattet das Einbringen auch von hochviskosen Pasten in eine Zahnfleischfurche (sulcus gingivae). Durch die Abrundung des Auslassendes kann die Gefahr einer Verletzung des Zahnfleisches verringert werden.

Der biegsame Bereich der Kanüle weist erfindungsgemäß eine Länge von 15 *mm* bis 25 *mm*, vorzugsweise ca. 20 *mm* auf. Eine entsprechende Länge ist ausreichend, um die gewünschte Biegsamkeit ohne Knicken zu erreichen. Gleichzeitig wird sichergestellt, dass die Spitze der Kanüle nicht unnötig weit aus der Vorrichtung zum Ausbringen der Dentalmasse - bspw. einen Standarddispenser - hervorsteht, was die Handhabung erschweren könnte.

Vorzugsweise ist der Behälter im Wesentlichen rohrförmig, hat einen Außendurchmesser von 6 *mm* bis 7 *mm,* vorzugsweise von 6,2 *mm* bis 6,6 *mm,* weist eine Wandstärke von wenigstens 0,5 *mm*, vorzugsweise wenigstens 0,9 *mm* und/oder eine Wandstärke kleiner als 1,5 *mm*, vorzugsweise kleiner als 1,0 *mm* auf. Die genannten Eigenschaften des Behälters sind dabei beliebig miteinander kombinierbar.

Der Behälter weist vorzugsweise im Bereich der Auslassöffnung und/oder der Aktuatoröffnung eine ringförmige Erweiterung des Außendurchmessers auf. Durch entsprechende Erweiterung(en) kann die Stabilität der Kanüle erhöht werden.

Die erfindungsgemäße Kartusche eignet sich für hochviskose Pasten mit einer dynamischen Viskosität von über 27.000 *Pas,* vorzugsweise bei einer Scherspannung von 9.000 *N*/*m*₂*,* einer Schergeschwindigkeit von 0,3 1/*s* und einer Temperatur von 23°*C*. Insbesondere lassen sich wasserhaltige adstringierende Retraktionspasten enthaltend Tonmineralien und korrosive Adstringenzien (z.B. 20% Wasser, 64% Tonmineral (bspw. Kaolin), 15% AlCl3x6H2O und 1% Additiven) lagern, ausgeben und auftragen. Die für das Ausgeben und Auftragen erforderliche Auspresskraft kann dabei auf unter 200 *N* begrenzt werden. Die Kartusche umfasst daher vorzugsweise eine in der Hohlkammer des Behälters angeordnete Dentalmasse, wobei die Dentalmasse vorzugsweise eine adstringierende Retraktionspaste - wie z.B. die vorstehend beschriebene Paste - ist. Der neben einer Flüssigkeit vorhandene Anteil der Füllstoffe kann bei der Dentalmasse größer 50 Gewichts-% sein. Die Dentalmasse kann eine starke Säure (*pKₛ* ≤ 2), insbesondere HCl, enthalten.

Die Erfindung wird nun anhand einer vorteilhaften Ausführungsform unter Bezugnahme auf die beigefügten Zeichnungen beispielhaft beschrieben. Es zeigen:
- Figur 1:: ein erstes Ausführungsbeispiel einer erfindungsgemäßen Kartusche;
- Figur 2:: eine Schnittansicht der Kartusche gemäß Figur 1; und
- Figur 3:: eine schematische Darstellung des manuellen Biegens der Kanüle der Kartusche gemäß Figuren 1 und 2.

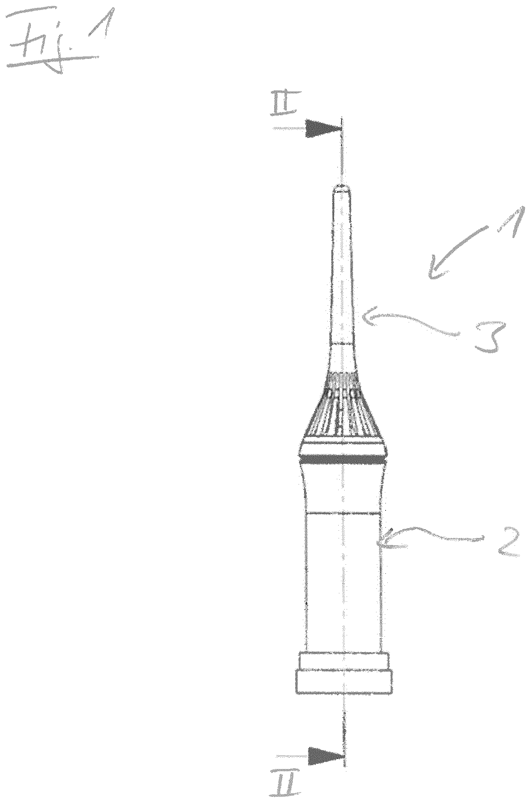

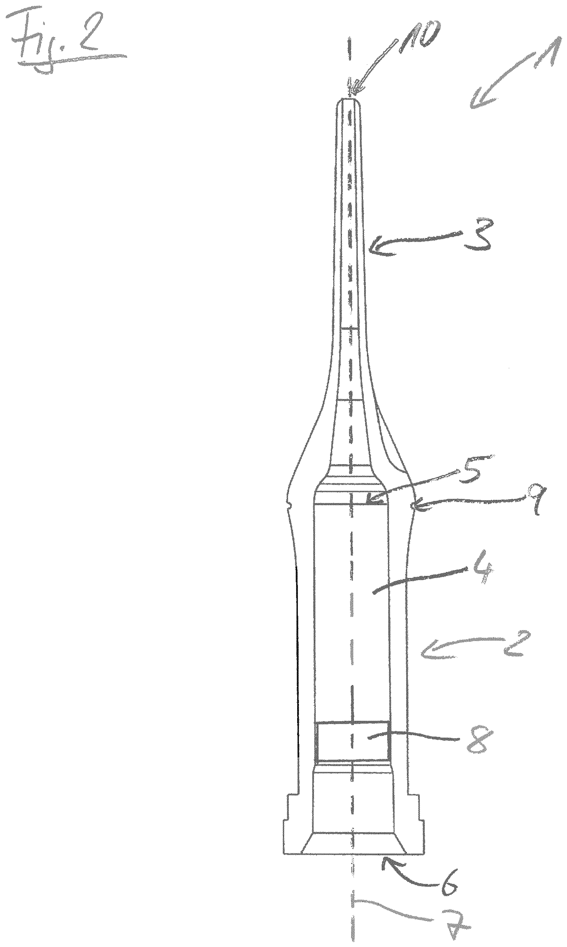

In Figuren 1 und 2 ist ein erstes Ausführungsbeispiel einer erfindungsgemäßen Kartusche 1 dargestellt. Die Kartusche 1 umfasst einen Behälter 2 und eine Kanüle 3, wobei die Kanüle 3 unlösbar mit dem Behälter 2 verbunden ist. Im dargestellten Ausführungsbeispiel sind Behälter 2 und Kanüle 3 einstückig miteinander ausgebildet und in einem Arbeitsgang durch 1-Kompenten-Spritzgießen hergestellt. Der für das dargestellte Ausführungsbeispiel verwendete Kunststoff für die Herstellung der Kanüle 1 ist Acrylnitril-Butadien-Styrol (ABS) mit einem Biegemodul von 2,4 *GPa* bei 23°*C*.

Der Behälter 2 ist rohrförmig ausgestaltet und umfasst eine Hohlkammer 4 zur Aufnahme einer Dentalmasse (nicht dargestellt). Die Hohlkammer 4 wiederum umfasst eine Auslassöffnung 5 und eine Aktuatoröffnung 6, die auf einer gemeinsamen Längsachse 7 angeordnet sind. In der Hohlkammer 4 ist ein Kolben 8 angeordnet, der im dargestellten Ausgangszustand der Kartusche 1 nahe der Aktuatoröffnung 6 angeordnet ist, sodass die in der Hohlkammer 8 befindliche Dentalmasse zwischen Kolben 8 und Auslassöffnung 5 aufgenommen werden kann.

Der Behälter 2 hat einen Außendurchmesser von 6,5 *mm* bei einer mittleren Wandstärke von 2 *mm.* Außerdem weist der Behälter 2 ringförmige Erweiterungen des Außendurchmessers 9 jeweils im Bereich der Auslassöffnung 5 und der Aktuatoröffnung 6 auf. Aufgrund dieser Ausgestaltung des Behälters 2 ist unmittelbar ersichtlich, dass sich die Kartusche 1 in einem Standarddispenser (nicht dargestellt) verwenden lässt.

Die Kanüle 3 ist derart am Behälter 2 angeformt, dass sie mit der Auslassöffnung 5 fluidverbunden ist, damit aus der Auslassöffnung 5 des Behälters 2 austretende Dentalmasse unmittelbar in die Kanüle 3 gelangt. In dem in Figuren 1 und 2 dargestellten Ausgangszustand verläuft die Kanüle 3 entlang der Achse 7 und weist eine Länge von insgesamt 25 *mm* auf, wobei deren nachfolgend beschriebene biegsame Bereich 3 ausgehend von der Auslassöffnung 10 der Kanüle 3 über 20 *mm* erstreckt. Die Auslassöffnung 10 der Kanüle 3 weist einen Innendurchmesser von 0,7 *mm* auf und ist mit einem Radius von 0,1 *mm* abgerundet.

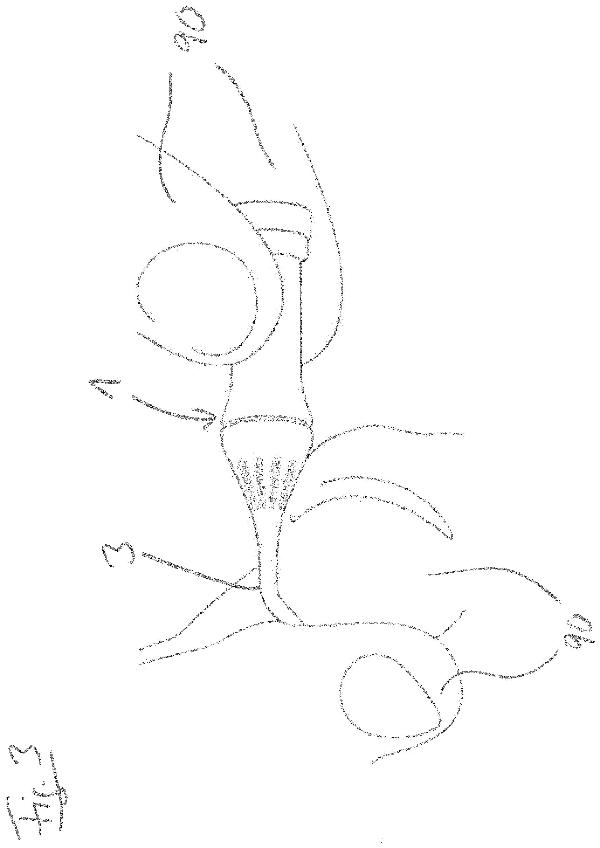

Die Kanüle 3 ist bei Standardbedingungen allein unter manueller Kraftaufwendung plastisch verformbar, sodass sich gegenüber dem in Figuren 1 und 2 gezeigten Ausgangszustand eine dauerhafte Biegung um einen Winkel von 65° ohne Knicken der Kanüle 3 erreichen lässt. In Figur 3 ist beispielhaft gezeigt, wie die Kanüle 3 der Kartusche 1 manuell bzw. allein mit Fingern 90 gebogen werden kann. Die Biegung ist dabei dauerhaft, bleibt also auch bestehen, wenn die Finger 90 weggenommen werden. Gleichzeitig erfolgt das Biegen bis zu 65° vollständig ohne Knicken, d.h. es gibt über die gesamte Länge der Kanüle 3 zu keinen maßgeblichen Formänderungen, bspw. durch Ausbeulen. Lediglich die Fläche des Innenquerschnitts der Kanüle 3, die sich im Ausgangszustand aus dem Innendurchmesser ergibt, kann sich durch das Biegen - je nach Biegewinkel - um bis zu maximal 5% verringern.

Die Kartusche 1 eignet sich für hochviskose Dentalmassen mit einer dynamischen Viskosität von über 27.000 *Pas* bei einer Scherspannung von 9.000 *N*/*m*₂, einer Schergeschwindigkeit von 0,3 1/*s* und einer Temperatur von 23°*C*. Insbesondere kann es sich bei der Dentalmasse um eine wasser-haltige adstringierende Retraktionspaste enthaltend Tonmineralien und korrosive Adstringenzien handeln, deren Anteil an Füllstoffen größer 50 Gewichts-% ist und HCl als starke Säure (*pKₛ* ≤ 2) enthält. Indem auch die Kanüle 3 bei der erfindungsgemäßen Kartusche 1 ohne Metall auskommt, werden Korrosionsprobleme vermieden.

Wie bereits angesprochen, ist die in Figuren 1 bis 3 dargestellte Kartusche 1 für Standarddispenser ausgebildet, die eine maximale Auspresskraft von 200 *N* aufbringen können. Die Kanüle 3 ist dabei in ihrer Form, aber auch über das verwendete Material (siehe oben) an die vorstehend beschriebene Retraktionspaste angepasst, dass beim Ausbringen der hochviskosen Dentalmasse sich die gebogene Kanüle 3 (vgl. Figur 3) um maximal 1° zurückstellt. In anderen Worten bleibt also die zuvor manuell hergestellte Biegung der Kanüle 3 auch während des Ausbringens von Dentalmasse durch die Kanüle 3 im Wesentlichen formstabil.

## Patentansprüche

1. Kartusche (1) zum Lagern, Ausgeben und Auftragen einer Dentalmasse umfassend einen Behälter (2) mit einer Hohlkammer (4) zur Aufnahme der Dentalmasse und eine Kanüle (3), wobei die Hohlkammer (4) eine Auslassöffnung (5) und eine der Auslassöffnung (5) gegenüberliegende Aktuatoröffnung (6) umfasst, wobei in der Hohlkammer (4) ein Kolben (8) vorgesehen ist, der im Ausgangszustand der Kartusche (1) eine Hohlkammer (4) zwischen Kolben (8) und Auslassöffnung (5) bildet, die im Wesentlichen durch eine pastöse Dentalmasse ausgefüllt ist, und die Kanüle (3) fluidverbunden mit der Auslassöffnung (5) an dem Behälter (2) angeordnet und im Ausgangszustand in gerader Richtung verlaufend ist, **dadurch gekennzeichnet, dass**
die Kanüle (3) einstückig mit dem Behälter (2) ausgebildet ist, wobei die Kanüle (3) und der Behälter (2) aus thermoplastischem Kunststoff sind, die Auslassöffnung (10) der Kanüle (3) einen Innendurchmesser von 0,4 *mm* bis 2 *mm* aufweist, der biegsame Bereich der Kanüle (3) eine Länge von 15 *mm* bis 25 *mm* hat und die Kanüle (3) bei Standardbedingungen unter manueller Kraftaufwendung derart plastisch verformbar ist, dass sich gegenüber dem Ausgangszustand eine dauerhafte Biegung um einen Winkel von 65° ohne Knicken der Kanüle (3) erreichen lässt, wobei die Biegung der Kanüle (3) beim Ausbringen einer hochviskose Pasten aus der Hohlkammer (4) mit einer dynamischen Viskosität von über 27.000 *Pa s* bei einer Scherspannung von 9.000 *N*/*m*₂*,* einer Schergeschwindigkeit von 0,3 1/*s* und einer Temperatur von 23°*C* mit einer Auspresskraft von weniger als 130 *N* um höchstens 10° zurückgestellt wird.

2. Kartusche nach Anspruch 1,
**dadurch gekennzeichnet, dass**
der Kunststoff der Kanüle (3) gemäß DIN EN ISO 178:2013 bei 23°*C* ein Biegemodul von 2,2 bis 2,9 *GPa,* vorzugsweise von 2,3 bis 2,6 *GPa,* vorzugsweise von ca. 2,4 *GPa* aufweist.

3. Kartusche nach einem der vorhergehenden Ansprüche,
**dadurch gekennzeichnet, dass**
die Auslassöffnung (5) und die Aktuatoröffnung (6) der Hohlkammer (4) auf einer gemeinsamen Achse (7) liegen, wobei vorzugsweise die Kanüle (3) im Ausgangszustand parallel zu oder entlang der Achse (7) verläuft.

4. Kartusche nach einem der vorhergehenden Ansprüche,
**dadurch gekennzeichnet, dass**
die Kanüle (3) zusammen mit dem Behälter (2) durch 1-Komponenten- oder Mehrkomponenten-Spritzgießen hergestellt ist.

5. Kartusche nach einem der vorhergehenden Ansprüche,
**dadurch gekennzeichnet, dass**
die Kanüle (3) und der Behälter (2) aus einem im Wesentlichen amorphen thermoplastischen Copolymer, Terpolymer und/oder Quadropolymer, weiter vorzugsweise einem teilvernetzten im Wesentlichen amorphen thermoplastischen Copolymer, Terpolymer oder Quadropolymer, weiter vorzugsweise aus Acrylnitril-Butadien-Styrol (ABS) sind.

6. Kartusche nach einem der vorhergehenden Ansprüche,
**dadurch gekennzeichnet, dass**
der thermoplastische Kunststoff Pigmente und/oder Füllstoffe enthält, wobei der Anteil der Pigmente und/oder Füllstoffe vorzugsweise weniger als 50 Gewichts-%, weiter vorzugsweise weniger als 30 Gewichts-% beträgt.

7. Kartusche nach einem der vorhergehenden Ansprüche,
**dadurch gekennzeichnet, dass**
die Auslassöffnung (10) der Kanüle (3) einen Innendurchmesser von 0,6 *mm* bis 0,9 *mm* aufweist, wobei das Auslassende der Kanüle (3) vorzugsweise abgerundet ist, bevorzugt mit einem Radius von 0,5 *mm* bis 1,5 *mm*.

8. Kartusche nach einem der vorhergehenden Ansprüche,
**dadurch gekennzeichnet, dass**
der biegsame Bereich der Kanüle (3) eine Länge ca. 20 *mm* aufweist.

9. Kartusche nach einem der vorhergehenden Ansprüche,
**dadurch gekennzeichnet, dass**
der Behälter (2) im Wesentlichen rohrförmig ist, einen Außendurchmesser von 6 *mm* bis 7 *mm*, vorzugsweise von 6,2 *mm* bis 6,6 *mm* hat, eine Wandstärke von wenigstens 0,5 *mm*, vorzugsweise wenigstens 0,9 *mm* und/oder eine Wandstärke kleiner als 1,5 *mm*, vorzugsweise kleiner als 1,0 *mm* aufweist.

10. Kartusche nach einem der vorhergehenden Ansprüche,
**dadurch gekennzeichnet, dass**
der Behälter (2) im Bereich der Auslassöffnung (5) und/oder der Aktuatoröffnung (6) eine ringförmige Erweiterung des Außendurchmessers (9) aufweist.

11. Kartusche nach einem der vorhergehenden Ansprüche,
**dadurch gekennzeichnet, dass**
die Dentalmasse in der Hohlkammer (4) des Behälters (2) eine adstringierende Retraktionspaste ist.

12. Kartusche nach einem der vorhergehenden Ansprüche,
**dadurch gekennzeichnet, dass**
die Dentalmasse eine Flüssigkeit und einen Anteil Füllstoffe von größer 50 Gewichts-% aufweist.

13. Kartusche nach einem der vorhergehenden Ansprüche,
**dadurch gekennzeichnet, dass**
die Dentalmasse eine starke Säure (*pKₛ* ≤ 2), insbesondere HCl, enthält.

## Claims

1. Cartridge (1) for storing, discharging and applying a dental compound, comprising a container (2), which has a hollow chamber (4) for accommodating the dental compound, and a cannula (3), wherein the hollow chamber (4) comprises an outlet opening (5) and an actuator opening (6) opposite the outlet opening (5), wherein a piston (8) is provided in the hollow chamber (4), said piston (8) forming, in the initial state of the cartridge (1), a hollow chamber (4) between piston (8) and outlet opening (5), which hollow chamber (4) is filled substantially by a pasty dental compound, and the cannula (3) is arranged on the container (2) in such a way as to be fluidically connected to the outlet opening (5) and is straight in the initial state, **characterized in that**
the cannula (3) is formed integrally with the container (2), wherein the cannula (3) and the container (2) are made from thermoplastic, the outlet opening (10) of the cannula (3) has an inside diameter of from 0.4 mm to 2 mm, the flexible region of the cannula (3) has a length of from 15 mm to 25 mm, and the cannula (3) is plastically deformable in such a manner under standard conditions, with the application of manual force, that a permanent bend by an angle of 65° in comparison with the initial state can be achieved without buckling of the cannula (3), wherein the bend in the cannula (3) is reversed by at most 10° as a highly viscous paste is dispensed from the hollow chamber (4) with a dynamic viscosity of over 27 000 Pa s, at a shear stress of 9000 N/m², a shear rate of 0.3 1/s and a temperature of 23°C with a pressing force of less than 130 N.

2. Cartridge according to Claim 1, **characterized in that**, at 23°C, the plastic of the cannula (3) has a bending modulus of from 2.2 to 2.9 GPa, preferably of from 2.3 to 2.6 GPa, preferably of about 2.4 GPa, in accordance with DIN EN ISO 178:2013.

3. Cartridge according to either of the preceding claims, **characterized in that** the outlet opening (5) and the actuator opening (6) of the hollow chamber (4) lie on a common axis (7), wherein the cannula (3) preferably extends parallel to or along the axis (7) in the initial state.

4. Cartridge according to one of the preceding claims, **characterized in that** the cannula (3) is produced together with the container (2) by 1-component or multicomponent injection moulding.

5. Cartridge according to one of the preceding claims, **characterized in that** the cannula (3) and the container (2) are made from a substantially amorphous thermoplastic copolymer, terpolymer and/or quadropolymer, further preferably a partially cross-linked, substantially amorphous thermoplastic copolymer, terpolymer or quadropolymer, further preferably from acrylonitrile butadiene styrene (ABS).

6. Cartridge according to one of the preceding claims, **characterized in that** the thermoplastic contains pigments and/or fillers, wherein the proportion of pigments and/or fillers is preferably less than 50% by weight, further preferably less than 30% by weight.

7. Cartridge according to one of the preceding claims, **characterized in that** the outlet opening (10) of the cannula (3) has an inside diameter of from 0.6 mm to 0.9 mm, wherein the outlet end of the cannula (3) is preferably rounded, preferably with a radius of from 0.5 mm to 1.5 mm.

8. Cartridge according to one of the preceding claims, **characterized in that** the flexible region of the cannula (3) has a length of about 20 mm.

9. Cartridge according to one of the preceding claims, **characterized in that** the container (2) is substantially tubular, has an outside diameter of from 6 mm to 7 mm, preferably of from 6.2 mm to 6.6 mm, and has a wall thickness of at least 0.5 mm, preferably at least 0.9 mm and/or a wall thickness of less than 1.5 mm, preferably less than 1.0 mm.

10. Cartridge according to one of the preceding claims, **characterized in that** the container (2) has an annular enlargement of the outside diameter (9) in the region of the outlet opening (5) and/or of the actuator opening (6).

11. Cartridge according to one of the preceding claims, **characterized in that** the dental compound in the hollow chamber (4) of the container (2) is an astringent retraction paste.

12. Cartridge according to one of the preceding claims, **characterized in that** the dental compound comprises a liquid and a proportion of fillers greater than 50% by weight.

13. Cartridge according to one of the preceding claims, **characterized in that** the dental compound contains a strong acid (*pKₛ* ≤ 2), in particular HCl.

## Revendications

1. Cartouche (1) destinée à stocker, décharger et appliquer une masse dentaire, comprenant un contenant (2) muni d'une chambre creuse (4) destinée à loger la masse dentaire et une canule (3), la chambre creuse (4) comprenant une ouverture de sortie (5) et une ouverture d'actionneur (6) opposée à l'ouverture de sortie (5), un piston (8) étant prévu dans la chambre creuse (4), qui forme dans l'état initial de la cartouche (1) une chambre creuse (4) entre le piston (8) et l'ouverture de sortie (5), qui est essentiellement remplie par une masse dentaire pâteuse, et la canule (3) étant agencée en liaison fluidique avec l'ouverture de sortie (5) sur le contenant (2) et s'étendant dans l'état initial en direction droite,
**caractérisée en ce que**
la canule (3) est configurée d'un seul tenant avec le contenant (2), la canule (3) et le contenant (2) étant en matière plastique thermoplastique, l'ouverture de sortie (10) de la canule (3) présente un diamètre intérieur de 0,4 mm à 2 mm, la zone flexible de la canule (3) a une longueur de 15 mm à 25 mm, et la canule (3) est déformable plastiquement en conditions standard sous application de force manuelle de telle sorte qu'elle puisse atteindre, par rapport à l'état initial, une flexion durable d'un angle de 65° sans pliage de la canule (3), la flexion de la canule (3) étant réinitialisée d'au plus 10° lors de la distribution d'une pâte hautement visqueuse à partir de la chambre creuse (4) ayant une viscosité dynamique de plus de 27 000 Pa s à une contrainte de cisaillement de 9 000 N/m², une vitesse de cisaillement de 0,3 1/s et une température de 23 °C avec une force d'expression de moins de 130 N.

2. Cartouche selon la revendication 1, **caractérisée en ce que** la matière plastique de la canule (3) présente, selon DIN EN ISO 178:2013 à 23 °C, un module de flexion de 2,2 à 2,9 GPa, de préférence de 2,3 à 2,6 GPa, de préférence d'environ 2,4 GPa.

3. Cartouche selon l'une quelconque des revendications précédentes, **caractérisée en ce que** l'ouverture de sortie (5) et l'ouverture d'actionneur (6) de la chambre creuse (4) sont situées sur un axe commun (7), la canule (3) s'étendant de préférence parallèlement à ou le long de l'axe (7) dans l'état initial.

4. Cartouche selon l'une quelconque des revendications précédentes, **caractérisée en ce que** la canule (3) est fabriquée conjointement avec le contenant (2) par moulage par injection à un composant ou à plusieurs composants.

5. Cartouche selon l'une quelconque des revendications précédentes, **caractérisée en ce que** la canule (3) et le contenant (2) sont en un copolymère, terpolymère et/ou quadripolymère thermoplastique essentiellement amorphe, de manière davantage préférée en un copolymère, terpolymère ou quadripolymère thermoplastique essentiellement amorphe partiellement réticulé, de manière davantage préférée en acrylonitrile-butadiène-styrène (ABS).

6. Cartouche selon l'une quelconque des revendications précédentes, **caractérisée en ce que** la matière plastique thermoplastique contient des pigments et/ou des charges, la proportion des pigments et/ou des charges étant de préférence inférieure à 50 % en poids, de manière davantage préférée inférieure à 30 % en poids.

7. Cartouche selon l'une quelconque des revendications précédentes, **caractérisée en ce que** l'ouverture de sortie (10) de la canule (3) présente un diamètre intérieur de 0,6 mm à 0,9 mm, l'extrémité de sortie de la canule (3) étant de préférence arrondie, avantageusement avec un rayon de 0,5 mm à 1,5 mm.

8. Cartouche selon l'une quelconque des revendications précédentes, **caractérisée en ce que** la zone flexible de la canule (3) présente une longueur d'environ 20 mm.

9. Cartouche selon l'une quelconque des revendications précédentes, **caractérisée en ce que** le contenant (2) est essentiellement tubulaire, a un diamètre extérieur de 6 mm à 7 mm, de préférence de 6,2 mm à 6,6 mm, présente une épaisseur de paroi d'au moins 0,5 mm, de préférence d'au moins 0,9 mm, et/ou une épaisseur de paroi inférieure à 1,5 mm, de préférence inférieure à 1,0 mm.

10. Cartouche selon l'une quelconque des revendications précédentes, **caractérisée en ce que** le contenant (2) présente, dans la zone de l'ouverture de sortie (5) et/ou de l'ouverture d'actionneur (6), un élargissement annulaire du diamètre extérieur (9).

11. Cartouche selon l'une quelconque des revendications précédentes, **caractérisée en ce que** la masse dentaire dans la chambre creuse (4) du contenant (2) est une pâte de rétraction astringente.

12. Cartouche selon l'une quelconque des revendications précédentes, **caractérisée en ce que** la masse dentaire comprend un liquide et une proportion de charges de plus de 50 % en poids.

13. Cartouche selon l'une quelconque des revendications précédentes, **caractérisée en ce que** la masse dentaire contient un acide fort (pKₛ ≤ 2), notamment HCl.
